# FASCICULE DE BREVET EUROPEEN

(11) **EP 3 143 985 B1**
(45) Date de publication et mention de la délivrance du brevet: **14.08.2019**
(21) Numéro de dépôt: 16196203.0
(22) Date de dépôt: 04.03.2010
(51) Int. Cl.: A61K 8/99, A61Q 19/00

(54) **UTILISATION DE MICROORGANISMES PROBIOTIQUES POUR LIMITER LES IRRITATIONS CUTANEES**
EINSATZ VON PROBIOTISCHEN MIKROORGANISMEN ZUR EINSCHRÄNKUNG VON HAUTIRRITATIONEN
USE OF PROBIOTIC MICROORGANISMS TO REDUCE SKIN IRRITATION

(30) Priorité: 04.03.2009 FR 0951362; 19.03.2009 US 202627 P
(43) Date de publication de la demande: 22.03.2017
(62) Demande divisionnaire de: 10155429.3
(73) Titulaire: L'OREAL, 75008 Paris (FR); Société des Produits Nestlé S.A., 1800 Vevey (CH)
(72) Inventeur: GUENICHE, Audrey, 92500 RUEIL MALMAISON (FR)
(74) Mandataire: Nony

(56) Documents cités:
- EP-A- 1 609 463
- WO-A-02/28402
- WO-A-03/068250
- WO-A-2005/030230
- KALLIOMAKI ET AL: "Probiotics in primary prevention of atopic disease: a randomised placebo-controlled trial", LANCET THE, LANCET LIMITED. LONDON, GB, vol. 357, no. 9262, 7 avril 2001 (2001-04-07), pages 1076-1079, XP005061313, ISSN: 0140-6736
- ISOLAURI E ET AL: "PROBIOTICS IN THE MANAGEMENT OF ATOPIC ECZEMA", CLINICAL AND EXPERIMENTAL ALLERGY, BLACKWELL SCIENTIFIC PUBLICATIONS, LONDON, GB, vol. 30, 1 novembre 2000 (2000-11-01), pages 1604-1610, XP001064231, ISSN: 0954-7894

## Description

La présente invention vise à proposer un nouvel actif pour la prévention et/ou le traitement des irritations cutanées.

La peau humaine est constituée de deux compartiments, à savoir un compartiment profond, le derme et un compartiment superficiel, l'épiderme.

Elle constitue une barrière contre les agressions extérieures, notamment chimiques, mécaniques ou infectieuses, et, à ce titre, un certain nombre de réactions de défense contre les facteurs environnementaux (climat, rayons ultraviolets, tabac, etc.) et/ou les xénobiotiques, comme par exemple les micro-organismes, se produisent à son niveau. Cette propriété, appelée fonction barrière, est principalement assurée par la couche la plus superficielle de l'épiderme, à savoir la couche cornée, appelée *stratum corneum.*

Il est manifeste que la qualité de la barrière cutanée et des muqueuses est quotidiennement affectée suite à des agressions externes de type agents irritants (détergents, acides, bases, oxydants, réducteurs, solvants concentrés, gaz ou fumées toxiques), sollicitations mécaniques (frottements, chocs, abrasion, arrachement de la surface, projection de poussières, de particules, rasage ou épilation), ou déséquilibres thermiques ou climatiques (froid, sécheresse).

L'irritation cutanée est classiquement définie comme une réaction inflammatoire locale, réversible et non immunologique, caractérisée par un oedème et un érythème induit après un contact simple ou répété d'une substance chimique avec la peau.

Des substances appartenant à différentes familles de produits chimiques très différents comme les solvants kératiniques, les agents déshydratants ou les agents oxydants ou réducteurs peuvent être considérés comme des irritants.

L'irritation de la peau est un phénomène très important puisqu'elle représente approximativement entre 60 % et 80 % des cas cliniques de dermatites de contact non allergiques. La majorité des autres cas représentent des dermatites allergiques de contact.

La dermatite irritante de contact (DIC) est une maladie multifactorielle dont le déclenchement dépend à la fois de facteurs intrinsèques et extrinsèques. L'âge, le fond génétique, le sexe sont autant de facteurs qui peuvent influer sur le développement de cette pathologie.

La dermatite irritante de contact (DIC) aiguë est principalement caractérisée par une inflammation alors que la DIC chronique est caractérisée par une hyper-prolifération des kératinocytes et une hyperkératose transitoire.

Les évènements biochimiques impliqués dans l'irritation cutanée sont complexes et très peu décrits.

On sait que l'irritation cutanée met en oeuvre une cascade de réactions qui, par le recrutement de cellules sanguines infiltrantes (neutrophiles, macrophages, cellules de Langherans) et les substances qu'elles libèrent (cytokines, lymphokines, chemokines...), donne naissance au processus irritant persistant qui se caractérise principalement par une irritation de la peau.

La pénétration cutanée des composés chimiques est un paramètre majeur dans l'établissement de la physiopathologie de la D1C. Celle-ci est liée au degré de perméabilité de la peau (qui est lié à son état physiologique) et aux propriétés physicochimiques des composés dont elle est supposée restreindre l'entrée (poids moléculaire, polarité, stade d'ionisation) et à la nature de l'environnement (excipient, véhicule) par lequel ces substances sont amenées au contact de la peau.

Cette étape indispensable correspond, à partir du milieu extérieur ou du véhicule, à la libération de la molécule qui va diffuser, et donc à sa mise à disposition de l'organisme.

Lors d'un contact entre un irritant et la peau, les kératinocytes sont les premières cellules à être activées par le produit chimique. La plupart des études sur la DIC se sont ainsi focalisées sur ce type cellulaire et de nombreuses données sont désormais connues quant à leur participation dans la physiopathologie de la DIC. Les kératinocytes jouent un rôle important dans l'initialisation de la réaction inflammatoire cutanée à travers la libération de nombreux médiateurs et de cytokines à l'origine de toute une cascade de l'inflammation aboutissant aux signes cliniques de la DIC. Parmi ceux-ci l'IL-la et les dérivés de l'acide arachidonique revêtent une importance particulière dans le développement de l'inflammation. Alors que le stress oxydatif joue un rôle majeur, celui du TNF-α semble plus controversé.

La libération de l'IL-la induit, via l'activation du facteur de transcription NF-kB, la transcription de gènes impliqués dans l'inflammation comme les cytokines IL-1α, IL-6, GM-CSF, le TNF-α, les chimiokines dont l'IL-8, MCP-1, MIP-1α et l'eotaxine, ainsi que l'expression de molécules d'adhésion comme l'E-selectine, l'ICAM-I et VCAM-I (Gordon JR, Nature 1990 :346 (6281) : 274-276).

La cascade de signalisation générée à partir de l'activation des kératinocytes commence à partir de la libération de médiateurs clés pré-stockés. En effet, les kératinocytes au repos contiennent de grande quantité d'IL-1α préformée et biologiquement active (Marks F et coll., Toxicol LeU 96 :111-118, 1998), ainsi que de l'acide arachidonique (Murphy JE et coll., J Invest Dermatol 114: 602-608, 2000).

Parce que ces deux composés sont constitutivement produits par les kératinocytes, et restent stockés dans la cellule, l'épiderme peut être considéré comme un grand réservoir de médiateurs hautement inflammatoire. Une altération des kératinocytes par l'effet corrosif d'un produit chimique, d'une brûlure ou par exposition aux UV, induit automatiquement la libération d'IL-1α et d'acide arachidonique qui deviennent les premiers évènements de défense de l'organisme.

Ainsi, l'IL-1α et l'acide arachidonique pourraient être considérés comme les médiateurs clés du déclenchement de l'irritation en réponse à un stress chimique (Murphy JE et coll., J Invest Dermatol, 114 : 602-608, 2000).

Parmi tous les médiateurs de l'inflammation, hormis l'IL-1 et l'acide arachidonique, seul le TNF-α peut activer un nombre suffisant de mécanismes pour générer indépendamment une inflammation cutanée. Cette cytokine majeure de l'inflammation cutanée est déjà pré-stockée dans les mastocytes dermiques (Larrick JW et coll., J Leukoc Biol, 45 :429-433, 1989), mais est également produite par les kératinocytes et les cellules de Langerhans après stimulation (Groves RW, et coll., J Invest Dermatol, 98 :384-387,1992). Un des mécanismes par lequel le TNF-α influence le plus la réaction inflammatoire est l'induction de molécules d'adhésion en synergie avec l'IL-1. Les molécules d'adhésion jouent un rôle essentiel dans la circulation et la pénétration des leucocytes (en particulier des neutrophiles) à partir des vaisseaux sanguins périphériques vers le derme et l'épiderme (Holliday MR et coll., Am J Contact Dermat, 8 :158-164, 1997).

De nombreux produits chimiques peuvent induire une irritation cutanée, cependant ils diffèrent dans leur capacité à générer des cytokines pro-inflammatoires, et une inflammation cutanée n'est pas systématiquement dépendante de la production de TNF-α.

Il est important de noter de plus, que la production d'IL-12 et d'IL-18 par les macrophages activés au site de l'inflammation joue un rôle important comme boucle d'amplification locale. En effet, ces cytokines stimulent la production d'IFN-α par les lymphocytes T avoisinants, qui, en retour, est un puissant facteur de co-activation des macrophages et des kératinocytes.

Enfin, pour ce qui est du stress exogène, on sait que des composés topiques peuvent, dans des circonstances particulières, conduire à l'apparition de réactions cutanées, lorsqu'ils sont utilisés dans des compositions cosmétiques ou dermatologiques, bien entendu pour d'autres effets.

Ainsi, on utilise des compositions cosmétiques contenant par exemple des actifs kératolytiques et/ou desquamants pour lutter contre le vieillissement, et notamment des actifs exfoliants et/ou des actifs favorisant le renouvellement cellulaire, tels que les α-hydroxy-acides (notamment les acides lactique, glycolique ou citrique), les β-hydroxy-acides (notamment les acides salicylique ou n-octanoyl-5-salicylique), et les rétinoïdes (notamment l'acide rétinoïque tout trans ou 13-cis et le rétinol). Malheureusement, si ces actifs sont utilisés en des quantités trop importantes, ils peuvent provoquer une irritation cutanée. De manière générale, les troubles cutanés évoqués précédemment sont plus fréquents dans les zones les plus exposées de l'organisme, à savoir les mains, les pieds, le visage, et le cuir chevelu.

Ils peuvent survenir notamment sur des zones soumises à certains gestes d'hygiène quotidienne ou fréquemment renouvelés tels que le rasage, l'épilation, la détergence par des produits de toilette ou des produits ménagers, l'application d'adhésifs (pansements, patchs, fixation de prothèses), ou dans le cas de gestes sportifs, professionnels ou simplement liés au mode de vie et à l'utilisation de vêtements, d'outils ou d'équipements générant des frottements localisés. Ils peuvent également être amplifiés par le stress psychologique.

En conséquence, il apparaît précieux de disposer d'un actif efficace pour prévenir et/ou traiter et/ou diminuer ces troubles, et plus particulièrement l'irritation cutanée.

Il a maintenant été constaté de manière tout à fait surprenante que l'utilisation d'un microorganisme du genre *Lactobacillus sp.* et/ou *Bifidobacterium species* ou l'une de leurs fractions permettait de donner satisfaction en ces termes.

Ainsi, selon un premier aspect, la présente description mentionne l'utilisation cosmétique d'une quantité efficace d'au moins un microorganisme probiotique notamment du genre *Lactobacillus sp.* et/ou *Bifidobacterium sp.,* de l'une de ses fractions et/ou de l'un de ses métabolites, à titre d'actif pour limiter une irritation cutanée.

Comme il ressort des exemples ci-après, les microorganismes considérés s'avèrent tout particulièrement utiles pour le maintien de mécanisme(s) favorisant la régulation des irritations cutanées.

La présente description mentionne également une quantité efficace d'au moins un microorganisme probiotique selon l'invention, notamment du genre *Lactobacillus sp.* et/ou *Bifidobacterium sp.,* de l'une de ses fractions et/ou de l'un de ses métabolites à titre d'actif pour la préparation d'une composition destinée à prévenir la manifestation et/ou traiter des troubles cutanés de type irritations.

La présente description rapporte également l'utilisation cosmétique, de préférence par voie topique, d'une quantité efficace d'au moins un microorganisme probiotique selon l'invention, notamment du genre *Lactobacillus sp.* et/ou *Bifidobacterium sp.,* et en particulier de la souche *Lactobacillus paracasei* ST11, de l'une de ses fractions et/ou de l'un de ses métabolites à titre d'actif destiné à prévenir la manifestation et/ou traiter les dermatites irritantes de contact (DIC).

La présente description concerne en outre selon un autre de ses aspects, un procédé de traitement cosmétique pour prévenir et/ou traiter les troubles cutanés du type irritation, notamment les peaux irritées, et en particulier la dermatite irritante de contact (DIC) comprenant l'administration, notamment orale ou topique, d'une quantité efficace d'au moins un microorganisme appartenant à l'espèce *Lactobacillus paracasei*, l'une de ses fractions, ou l'un de ses métabolites.

A la connaissance des inventeurs, l'efficacité d'un microorganisme probiotique du genre *Lactobacillus sp*, et en particulier de l'espèce *Lactobacillus paracasei*, de l'une de ses fractions et/ou de l'un de ses métabolites, n'a jamais été décrite.

La mise en oeuvre de microorganismes, notamment probiotiques, pour le soin de la peau a déjà été décrite.

Le document WO 2006/07922 décrit des compositions dédiées au traitement des peaux sensibles mettant en oeuvre, à titre d'actif, une association d'un microorganisme *Lactobacillus paracasei* ou *casei* et d'un microorganisme *Bifidobacterium longum* ou *Bifidobacterium lactis.*

FR 2 872 047 décrit, pour sa part, une association d'un microorganisme probiotique avec un cation minéral divalent.

FR 2 889 057, quant à lui, divulgue une composition topique, comprenant un microorganisme probiotique en association avec un acide gras polyinsaturé et/ou un ester d'acide gras polyinsaturé, utile par le traitement des peaux sensibles.

WO 02/28402 décrit la mise en oeuvre de microorganismes probiotiques pour réguler les réactions d'hypersensibilité cutanée, qui sont des réactions allergiques.

Enfin, WO 03/070260 concerne la mise en oeuvre de microorganismes probiotiques à des fins de photoprotection de la peau.

Toutefois, aucun de ces documents ne décrit la mise en oeuvre de microorganismes probiotiques selon l'invention, notamment du genre *Lactobacillus sp*., en particulier de l'espèce *Lactobacillus paracasei*, et en particulier de la souche *Lactobacillus paracasei* ST11, de l'une de ses fractions et/ou de l'un de ses métabolites en tant qu'actif utile à l'égard des troubles d'irritation cutanés.

Comme il ressort des essais présentés ci-après, il est constaté à l'issue d'une administration sur une période prolongée d'un microorganisme conforme à l'invention, une régulation des irritations cutanées, et notamment leur limitation.

En particulier, les inventeurs ont démontré qu'un tel microorganisme permet de stimuler sélectivement la production de la cytokine régulatrice IL-8.

Selon un premier aspect, l'invention concerne donc une utilisation cosmétique d'une quantité efficace d'au moins un microorganisme probiotique de l'espèce Lactobacillus paracasei, de l'une de ses fractions et/ou de l'un de ses métabolites, comme agent pour renforcer la protection de la peau vis-à-vis d'agression(s) externe(s) choisies parmi :
- des sollicitations mécaniques ; ou
- des déséquilibres thermiques ou climatiques.

Selon un second aspect, l'invention concerne une composition comprenant une quantité efficace d'au moins un microorganisme probiotique de l'espèce *Lactobacillus paracasei*, de l'une de ses fractions et/ou de l'un de ses métabolites, comme agent destiné à être utilisé pour renforcer la protection de la peau vis-à-vis d'agression(s) externe(s) d'agents irritants.

Ainsi, les compositions qui contiennent au moins un microorganisme conforme à l'invention peuvent être destinées à prévenir et/ou à diminuer la manifestation des signes cutanés de type irritation, en particulier induits par un stress exogène d'origine chimique, environnemental ou mécanique.

Une irritation cutanée peut notamment être induite par au moins une condition choisie parmi l'action de produits chimiques, de composés susceptibles de provoquer une irritation de la peau, de la température, du climat, de la pollution atmosphérique ou des frottements.

Selon une variante préférée, le stress considéré selon l'invention est différent de celui inhérent à une opération d'épilation ou de peeling.

Notamment, les microorganismes conformes à la présente invention peuvent être mis en oeuvre à titre d'agent anti-irritant.

L'irritation cutanée considérée dans l'invention ne met pas en oeuvre de composante immunologique, et à ce titre est distincte, par exemple du psoriasis, de l'hypersensibilité de contact ou de l'hypersensibilité retardée.

L'irritation cutanée considérée dans l'invention est en particulier une irritation cutanée non allergique. Plus particulièrement la présente invention vise à prévenir et/ou traiter la dermite irritante de contact non allergique.

L'invention concerne donc également, selon un autre de ses aspects, l'utilisation cosmétique d'au moins une quantité efficace d'au moins un microorganisme probiotique de l'espèce *Lactobacillus paracasei*, de l'une de ses fractions et/ou de l'un de ses métabolites, comme agent pour renforcer la protection de la peau vis-à-vis d'agressions extérieures, ou agression(s) externe(s).

En particulier, elle vise l'utilisation cosmétique d'une quantité efficace d'au moins un microorganisme probiotique de l'espèce *Lactobacillus paracasei*, de l'une de ses fractions et/ou de l'un de ses métabolites pour prévenir l'irritation cutanée susceptible d'être induite par une ou des agression(s) externe(s) de type sollicitation(s) mécanique(s) comme par exemple les frottements, les chocs, l'abrasion, l'arrachement de la surface, les projections de poussières, de particules, le rasage, ou à l'égard de déséquilibres thermique(s) ou climatique(s) comme par exemple le froid.

Ainsi, elle vise l'utilisation cosmétique d'une quantité efficace d'un lysat d'au moins un microorganisme probiotique de l'espèce *Lactobacillus paracasei*, de l'une de ses fractions et/ou de l'un de ses métabolites pour prévenir et/ou traiter les troubles cutanés - de type irritation consécutifs à une exposition aux polluants atmosphériques.

L'invention vise en outre l'utilisation cosmétique d'une quantité efficace d'au moins un microorganisme probiotique de l'espèce *Lactobacillus paracasei*, de l'une de ses fractions et/ou de l'un de ses métabolites pour prévenir et/ou traiter les troubles cutanés de type irritation générés par un stress oxydatif initié par un oxydant environnemental, tel que par exemple l'oxygène, l'ozone et/ou les oxydes d'azote et de soufre.

L'invention vise également des compositions comprenant une quantité efficace d'au moins un microorganisme probiotique de l'espèce *Lactobacillus paracasei*, de l'une de ses fractions et/ou de l'un de ses métabolites, comme agent destiné à être utilisé pour renforcer la protection de la peau vis-à-vis d'agression(s) externe(s) d'agents irritants, tels que par exemple les détergents, les acides, les bases, les oxydants, les réducteurs, les solvants concentrés, les gaz et fumées toxiques.

Par « quantité efficace », on entend au sens de la présente invention une quantité suffisante pour obtenir l'effet attendu.

Au sens de la présente invention, le terme « prévenir » signifie réduire le risque de manifestation du phénomène considéré.

Cette réduction peut conduire à un risque de degré moindre à celui préexistant à l'utilisation selon l'invention.

Au sens de la présente invention, le terme « traiter » signifie suppléer à un dysfonctionnement physiologique et plus généralement à diminuer, voire supprimer le désordre indésirable et dont la manifestation est notamment une conséquence de ce dysfonctionnement.

Au sens de l'invention, on entend par « limiter » au regard d'un symptôme, le fait de diminuer l'intensité d'expression de ce symptôme, par exemple une irritation cutanée, et/ou de diminuer le risque de survenue de ce symptôme.

Il est entendu que la diminution de l'intensité et/ou du risque considéré peut être totale ou partielle, i.e. le risque de survenue du symptôme ou l'intensité de son expression demeure mais à un degré moindre que celui préexistant à l'utilisation selon l'invention.

Selon une variante de réalisation de l'invention, un microorganisme selon l'invention peut être mis en oeuvre par voie orale.

Selon une autre variante de réalisation de l'invention, le microorganisme selon l'invention peut être mis en oeuvre par voie topique.

Les produits topiques agissent néanmoins, par définition, localement sur les zones à traiter, zones sur lesquelles ils peuvent être inégalement répartis, et nécessitent des applications soignées et répétées.

Par opposition, la voie orale présente l'avantage d'agir de façon globale sur l'ensemble de la peau et ce dans ses couches profondes (derme, hypoderme), suivant un mode d'administration rapide et peu contraignant. En effet, les métabolites et autres nutriments actifs sont en particulier distribués au sein de la matrice dermique par le biais de la circulation sanguine.

La voie orale ou l'administration par patch présentent également l'avantage d'un mode d'administration rapide et peu contraignant.

Selon un mode de réalisation préféré, l'utilisation cosmétique selon l'invention est donc effectuée par voie orale et le procédé selon l'invention comprend l'administration par voie orale de ladite quantité efficace du microorganisme considéré selon l'invention ou l'une de ses fractions, ou l'un de ses métabolites.

Comme précisé ci-après, les compositions contenant ledit microorganisme sont formulées pour être compatibles avec le mode d'administration retenu.
*rhamnosus (*souche GG*), Lactobacillus brevis, Lactobacillus crispatus, Lactobacillus delbrueckii (subsp bulgaricus, lactis), Lactobacillus fermentum, Lactobacillus helveticus, Lactobacillus gallinarum, Lactobacillus gasseri, Lactobacillus johnsonii (*CNCM I-1225*), Lactobacillus plantarum, Lactobacillus reuteri, Lactobacillus salivarius, Lactobacillus alimentarius, Lactobacillus curvatus, Lactobacillus casei subsp. casei, Lactobacillus sake, Lactococcus lactis, Enterococcus (faecalis, faecium), Lactococcus lactis (subspp lactis ou cremoris), Leuconostoc mesenteroides* subsp *dextranicum, Pediococcus acidilactici, Sporolactobacillus inulinus, Streptococcus salvarius subsp. thermophilus, Streptococcus thermophilus, Staphylococccus carnosus, Staphylococcus xylosus, Saccharomyces (cerevisiae* ou encore *boulardii), Bacillus (cereus var toyo* ou *subtilis), Bacillus coagulans, Bacillus licheniformis, Escherichia coli strain nissle, Propionibacterium freudenreichii,* et leurs mélanges.

Plus particulièrement, il peut s'agir de microorganismes probiotiques issus du groupe des bactéries lactiques, comme notamment les *Lactobacillus* et/ou les *Bifidobacterium.*

A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei, Bifidobacterium bifidum, Bifidobacterium breve, Bifidobacterium longum, Bifidobacterium animalis, Bifidobacterium lactis, Bifidobacterium infantis, Bifidobacterium adolescentis, Bifidobacterium pseudocatenulatum,* et leurs mélanges.

Les espèces convenant tout particulièrement sont les *Lactobacillus johnsonii, Lactobacillus paracasei, Bifidobacterium adolescentis, Bifidobacterium longum* respectivement déposés suivant le traité de Budapest avec l'Institut Pasteur (28 rue du Docteur Roux, F-75024 Paris cedex 15) les 30/06/92, 12/01/99, 15/04/99 et le 15/04/99 sous les désignations suivantes CNCM 1-1225, CNCM 1-2116, CNCM 1-2168 et CNCM 1-2170, le *Bifidobacterium lactis (Bb 12)* (ATCC27536) ou le *Bifidobacterium longum (BB536).* La souche de *Bifidobacterium lactis* (ATCC27536) peut être obtenue chez Hansen (Chr. Hansen A/S, 10-12 Boege Alle, P.O. Box 407, DK-2970 Hoersholm, Danemark).

Selon un mode de réalisation, un microorganisme probiotique convenant à l'invention peut en particulier être un microorganisme du genre *Lactobacillus sp.*

De manière préférée, un microorganisme du genre *Lactobacillus sp.* convenant à l'invention peut être choisi parmi les espèces *Lactobacillus johnsonii, Lactobacillus reuteri, Lactobacillus rhamnosus, Lactobacillus paracasei, Lactobacillus casei,* et leurs mélanges.

Selon un mode de réalisation préféré, un microorganisme convenant à l'invention peut être un *Lactobacillus paracasei.*

Un microorganisme convenant à l'invention peut en particulier être la souche *Lactobacillus paracasei* déposée suivant le traité de Budapest auprès de l'Institut Pasteur (28 rue du Docteur Roux, F-75024 Paris cedex 15) le 12/01/99 sous la désignation CNCM I-2116, et/ou une de ses fractions et/ou un de ses métabolites.

Selon une variante de réalisation, l'invention concerne l'utilisation, outre d'un premier microorganisme probiotique, tel que défini ci-dessus, et notamment du genre *Lactobacillus* et/ou *Bifidobacterium sp.,* d'au moins une quantité efficace d'au moins un second microorganisme, notamment de type probiotique, et/ou une de ses fractions et/ou un de ses métabolites, distinct dudit premier microorganisme.

Ce second microorganisme peut être choisi notamment parmi les ascomycètes telles que *Saccharomyces, Yarrowia, Kluyveromyces, Torulaspora, Schizosaccharomyces pombe, Debaromyces, Candida, Pichia, Aspergillus* et *Penicillium,* les bactéries du genre *Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus, Lactobacillus, Bifidobacterium* et leurs mélanges.

Comme ascomycètes convenant tout particulièrement à la présente invention, on peut en particulier citer *Yarrowia lipolitica* et *Kluyveromyces lactis,* de même que *Saccharomyces cereviseae, Torulaspora, Schizosaccharamyces pombe, Candida* et *Pichia.*

Des exemples spécifiques de microorganismes probiotiques sont *Lactobacillus acidophilus, Lactobacillus alimentarius, Lactobacillus curvatus, Lactobacillus delbruckii subsp. lactis, Lactobacillus gasseri, Lactobacillus johnsonii, Lactobacillus reuteri, Lactobacillus rhamnosus (Lactobacillus GG), Lactobacillus sake, Lactococcus lactis, Streptococcus thermophilus, Staphylococccus carnosus, Staphylococcus xylosus,* et leurs mélanges.

Selon un mode de réalisation, à titre de second microorganisme ce sont les genres bactériens et de levure suivants qui sont préférentiellement utilisés :
- les bactéries lactiques, qui produisent par fermentation du sucre de l'acide lactique. Suivant leur morphologie on les divise en deux groupes :
   *Lactobacillus species : Lactobacillus acidophilus, amylovorus, casei, rhamnosus, brevis, crispatus, delbrueckii (subsp bulgaricus, lactis), fermentum, helveticus, gallinarum, gasseri, johnsonii, plantarum, reuteri, salivarius, alimentarius, curvatus, casei subsp. casei, sake, et*
   *Cocci : Enterococcus (faecalis, faecium), Lactococcus lactis (subsp lactis ou cremoris), Leuconostoc mesenteroides* subsp *dextranicum, Pediococcus acidilactici, Sporolactobacillus inulinus, Streptococcus salvarius subsp. thermophilus, Streptococcus thermophilus, Staphylococccus carnosus, Staphylococcus xylosus,*
- les bifidobactéries ou *Bifidobacterium species : Bifidobacterium adolescentis, animalis, bifidum, breve, lactis, longum, infantis, pseudocatenulatum,*
- les levures : *Saccharomyces (cerevisiae* ou encore *boulardii),*
- les autres bactéries sporulées : *Bacillus (cereus var toyo ou subtilis), Bacillus coagulans, Bacillus licheniformis, Escherichia coli strain nissle, Propionibacterium freudenreichii,*
- et leurs mélanges.

Plus particulièrement, il peut s'agir de l'un des microorganismes probiotiques proposés ci-dessus, à titre d'exemple spécifique de microorganismes probiotiques pour le premier microorganisme.

Selon un mode de réalisation particulier, le second microorganisme probiotique est du genre *Lactobacillus species,* en particulier de l'espèce *Lactobacillus johnsonii,* l'une de ses fractions et/ou l'un de ses métabolites.

Il peut notamment s'agir de l'espèce *Lactobacillus johnsonii* respectivement déposée suivant le traité de Budapest auprès de l'Institut Pasteur (28 rue du Docteur Roux, F-75024 Paris cedex 15) le 30/06/92, sous la désignation CNCM 1-1225.

Un microorganisme de l'invention peut être formulé dans une composition à raison d'au moins 0,0001 % (exprimé en poids sec), en particulier à raison de 0,0001 à 20 % et plus particulièrement à raison de 0,001 à 15 % en poids en particulier de 0,01 à 10 % en poids, et notamment de 0,1 % à 2 % en poids par rapport au poids total de la composition.

D'une manière générale, une composition selon l'invention et en particulier celle destinée à être administrée par voie orale peut comprendre pour les microorganismes vivants de 10³ à 10¹⁵ ufc/g, en particulier de 10⁵ à 10¹⁵ ufc/g et plus particulièrement de 10⁷ à 10¹² ufc/g de microorganismes par gramme de support ou à des doses équivalentes calculées pour les microorganismes inactifs ou morts ou pour des fractions de microorganisme ou pour des métabolites produits.

Dans le cas particulier des compositions devant être administrées par voie orale, la concentration en chaque microorganisme et/ou fraction et/ou métabolite correspondant peut être ajustée de manière à correspondre à des doses (exprimées en équivalent de microorganisme) variant de 5.10⁵ à 10¹³ ufc/j et en particulier de 10⁸ à 10¹¹ ufc/j.

Une composition à application topique selon l'invention peut comprendre généralement de 10³ à 10¹² ufc/g, en particulier de 10⁵ à 10¹⁰ ufc/g et plus particulièrement de 10⁷ à 10⁹ ufc/g de microorganismes.

Lorsqu'une composition comprend des métabolites, les teneurs en métabolites dans les compositions correspondent sensiblement aux teneurs susceptibles d'être produites par 10³ à 10¹⁵ ufc, en particulier 10⁵ à 10¹⁵ ufc, et plus particulièrement 10⁷ à 10¹² ufc de microorganismes vivants par gramme de support.

L'expression de la quantité de métabolites ou de fractions d'un microorganisme en « ufc » vise à désigner la quantité de ce microorganisme nécessaire à la production de ladite quantité de métabolites ou de fractions.

Le ou les microorganisme(s) peu(ven)t être inclus dans une composition selon l'invention sous une forme vivante, semi-active ou inactivée, morte.

Selon un mode de réalisation particulier, ces microorganismes sont mis en oeuvre sous une forme vivante.

Il(s) peu(ven)t également être inclus sous forme de fractions de composants cellulaires ou sous la forme de métabolites. Le ou le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Selon une variante, les compositions peuvent également contenir un cation minéral divalent.

Dans le cas particulier des compositions topiques, il peut être avantageux de mettre en oeuvre ces microorganismes sous forme inactivée, voire morte.

### COMPOSITION SELON LA PRESENTE INVENTION

Selon encore un autre de ses aspects, la présente invention a pour objet une composition notamment topique, cosmétique et/ou dermatologique, notamment utile pour prévenir, diminuer et/ou traiter un trouble cutané de type irritation, comprenant dans un support physiologiquement acceptable, au moins une quantité efficace d'au moins un microorganisme probiotique, notamment du genre *Lactobacillus sp.* et/ou *Bifidobacterium sp*., de l'une de ses fractions et/ou de l'un de ses métabolites, en association à une quantité efficace d'au moins un agent susceptible de provoquer une irritation de la peau.

Ainsi, selon un de ses aspects, la présente invention concerne l'utilisation d'un microorganisme de l'invention pour prévenir et/ou diminuer l'effet irritant d'une composition cosmétique ou dermatologique contenant un ou plusieurs composés susceptibles de provoquer une irritation de la peau.

Parmi ces composés on peut notamment citer des composés ou actifs cosmétiques, des composés ou actifs dermatologiques, des tensioactifs notamment des tensioactifs anioniques, des conservateurs, des détergents, des parfums et notamment des solutions alcooliques parfumantes, des solvants, des propulseurs, et leurs mélanges.

Parmi les autres agents irritants on peut citer : les acides pyruvique, gluconique, glucuronique, oxalique, malonique, succinique, acétique, gentisique, cinnamique, azélaique ; le phénol ; la résorcine ; l'urée et ses dérivés, l'hydroxyéthylurée ou Hydrovance® de chez NATIONAL STARCH ; les oligofucoses ; l'acide jasmonique et ses dérivés ; l'acide ascorbique et ses dérivés, l'acide trichloracétique ; l'extrait de Saphora japonica, et le résvératrol.

Les enzymes impliqués dans la desquamation ou la dégradation des cornéodesmosomes peuvent également être susceptibles de provoquer une irritation de la peau.

On peut également citer, à titre d'agents irritants, les agents chélatants des sels minéraux tels l'EDTA ; l'acide N-acyl-N,N',N'éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine tels que décrits dans EP 0 852 949, ainsi que le méthyl glycine diacétate de sodium commercialisé par BASF sous la dénomination commerciale TRILON M® ; le miel ; les dérivés de sucre tels que l'O-octanoyl-6-D-maltose, le O-linoleyl-6-D-glucose et la N-acétyl glucosamine.

Les rétinoïdes sont également des composés susceptibles de provoquer une irritation de la peau. On peut par exemple citer parmi eux le rétinol et ses esters, le rétinal, l'acide rétinoïque et ses dérivés tels que ceux décrits dans les documents FR-A-2 570 377, EP-A-0 199 636, EP-A-0 325 540, EP-A-0 402 072, et l'adapalène.

Les sels et dérivés, comme les formes cis ou trans, les mélanges racémiques, les formes dextrogyres ou levogyres des composés cités précédemment sont aussi considérés comme des composés susceptibles de provoquer une irritation de la peau.

D'autres actifs dermatologiques ou cosmétiques susceptibles de provoquer une irritation de la peau sont également cités ci-après :
- l'urée et ces dérivés comme l'hydroxyéthylurée ou Hydrovance® de NATIONAL STARCH ;
- certaines vitamines telles que la vitamine D et ses dérivés tels que la vitamine D3, la vitamine D2, le calcitriol, le calcipotriol, le tacalcitol, la 24,25-diOH vitamine D3, la 1-OH vitamine D2 et la 1,24-diOH vitamine D2 ; la vitamine B9 et ses dérivés ;
- les peroxydes comme le peroxyde de benzoyle, l'eau oxygénée,
- les antichutes tels que le minoxidil et ses derivés tel que l'aminexyl ;
- les teintures et les colorants capillaires comme les aminophénols et leurs dérivés tels que la para-phénylène diamine (p-PDA), la N-phenyl p-PDA, le toluène 2,5-diamine sulfate, la méta-phénylène diamine (m-PDA), la toluène 3,4-diamine et l'ortho-phénylène diamine (o-PDA) ;
- les agents anti-transpirants comme les sels d'aluminium, tel que l'hydroxychlorure d'aluminium ;
- les déodorants ;
- les actifs de permanentes tels que les thioglycolates, l'ammoniaque ;
- le thioglycolate et ses sels ;
- le phénoxyéthanol ;
- le 1,2-pentanediol ;
- les solutions alcooliques parfumantes (parfums, eaux de toilette, après rasage, déodorants) ;
- les anthralines (dioxyanthranol) ;
- les anthranoïdes (par exemple ceux décrits dans le document EP-A- 0 319 028),
- les sels de lithium ;
- les dépigmentants (ex : hydroquinone, vitamine C à forte concentration, acide kojique) ;
- certains actifs amincissants à effet chauffant ;
- les nicotinates et leurs dérivés ;
- la capsaïcine ;
- les actifs antipoux (pyréthrine) ;
- les antiprolifératifs tels que le 5-fluoro uracile ou le méthotrexate ;
- les agents antiviraux ;
- les antiparasitaires ;
- les antifongiques ;
- les antiprurigineux ;
- les antiséborrhéiques ;
- certaines filtres solaires ;
- les propigmentants tels que les psoralènes et les méthylangécilines ;
- et leurs mélanges.

Comme conservateurs, on peut citer le phénoxyéthanol, la chorehexidine et le chlorure de benzalkonium.

Comme tensioactifs, on peut citer les tensioactifs anioniques, cationiques et amphotères, plus particulièrement les tensioactifs anioniques tels que les alkyl sulfates et alkyl éther sulfates comme le lauryl sulfate et le lauryl éther sulfate, et leurs sels notamment de sodium.

Le composé plus particulièrement associé est choisi parmi les rétinoïdes, les α-hydroxyacides, les β-hydroxyacides, les acides dicarboxyliques saturés et insaturés tels que l'acide octadécène dioique ou Arlatone DIOC DCA vendu par la société Uniqema, les tensioactifs anioniques, cationiques ou amphotères, l'acide n-octanoyl 5-salicylique, les actifs antiperspirants tels que les sels d'aluminium, l'acide (N-2 hydroxyéthylpiperazine-N-2-éthane) sulfonique (HEPES) et l'acide cinnamique.

Le composé susceptible de provoquer une irritation de la peau peut être présent dans la composition selon la présente invention dans une quantité suffisante pour provoquer une réaction d'irritation de la peau. A titre d'exemple, il peut être présent dans une teneur allant de 0,0001 à 70 % en poids, de préférence de 0,01 à 50 % en poids et mieux de 0,1 à 30 % en poids par rapport au poids total de la composition.

### ACTIFS ADDITIONNELS

Les microorganismes considérés selon l'invention peuvent également être associé à au moins 0,00001 % à 95 % en poids d'un agent anti-inflammatoire, un autre agent apaisant ou leur mélange.

Comme exemples « d'agents anti-inflammatoires », on peut citer :
- un antagoniste de cytokines inflammatoires ;
- un anti-inflammatoire stéroïdien (hydrocortisone, betaméthasone, dexaméthasone etc) ;
- un anti-inflammatoire non stéroïdien comme l'aspirine ou le paracétamol ;
- et leurs mélanges.

Par « antagonistes de cytokines inflammatoires » selon l'invention, on entend un composé susceptible d'inhiber la synthèse et/ou la libération d'une ou plusieurs cytokines inflammatoires. Entrent également dans la définition d'un antagoniste des cytokines inflammatoires, les composés qui inhibent ou bloquent la fixation de ces cytokines sur leur(s) récepteur(s).

En particulier, l'autre agent apaisant pourra avantageusement être choisi parmi l'allantoïne, l'acide bêta-glycyrrhétinique, les extraits en contenant comme par exemple l'extrait de Glycyrrhiza Glabra (réglisse) et les complexes en contenant comme le complexe allantoïne/acide glycyrrhétinique; les planctons, lyophilisés ou non, leurs extraits et leurs complexes; les eaux et extraits de fleurs et de plantes: eaux de camomille, de tilleul, rose, extraits de bouleau; le bisabolol ; les huiles essentielles par exemple de coriandre ; les algues notamment du type *Laminaria* (par exemple rouges ou brunes) tel que l'extrait d'algue brune *Padina Pavonica* comme HPS 3 PADINA PAVONICA commercialisé par la société Alban Muller; l'acide acexamique et l'acide transexamique (acide trans-4, aminométhylcyclohexane carboxylique) ; l'acide ursolique et les extraits en contenant comme l'extrait de feuille de romarin; les polysaccharides contenant du fucose, comme le FUCOGEL 1000, vendu par la société Solabia; les électrolytes et en particulier un mélange aqueux comme le « mélange des sels de la mer Morte » (« Dead Sea Bath SaltS ») ; les acides aminés comme les Sepicalm S et VG de Seppic, et les sels divalents de magnésium tel que le magnésium gluconate.

Selon un mode de réalisation particulier de l'invention, l'agent anti-inflammatoire est choisi parmi les extraits d'algues capable de moduler la production des cytokines par les kératinocytes comme le Phycosaccharide® commercialisé par la société CODIF, l'extrait hydroglycolique de l'algue *Laminaria saccharina*, notamment la Phlorogine® commercialisée par la société SECMA, les extraits *d'Aloe vera*, l'extrait d'écorces et de racines de *terminalia sericea* ou SERICOSIDE 3058500 commercialisés par la société INDENA.

Les agents anti-inflammatoires sont de préférence présents dans les compositions conformes à l'invention à une concentration pouvant varier entre 0,00001 et 10 % en poids environ par rapport au poids total de la composition. Encore plus préférentiellement, la concentration en composé anti-inflammatoire peut varier entre 0,0005 % et 2 % en poids par rapport au poids total de la composition.

Dans les formes galéniques topiques, on peut utiliser plus particulièrement comme actifs hydrophiles les protéines ou les hydrolysats de protéine, les acides aminés, les polyols, notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Pour ce qui est des actifs lipophiles, on peut utiliser le rétinol (vitamine A) et ses dérivés, le tocophérol (vitamine E) et ses dérivés, les céramides, les huiles essentielles et les insaponifiables (tocotriénol, sésamine, gamma oryzanol, phytostérols, squalènes, cires, terpènes).

On peut également associer de façon avantageuse des actifs favorisant la desquamation comme les actifs hydratants de référence en cosmétique tel que le glycérol, l'acide hyaluronique, l'urée et ses dérivés ainsi que des actifs favorisant la desquamation comme les chélateurs, l'acide jasmonique et ses dérivés, particulièrement l'ER2412, les composés réducteurs, les dérivés sulfoniques et particulièrement l'Hepes, les acides aminés, les AHA et BHA, tout particulièrement l'acide glycolique et l'ER195, et certains détergents.

### FORMES GALENIQUES

Les compositions selon l'invention peuvent se présenter sous toutes les formes galéniques normalement disponibles pour le mode d'administration retenu.

Le support peut être de nature diverse selon le type de composition considérée. Les compositions destinées à une administration par voie topique, peuvent être des solutions aqueuses, hydroalcooliques ou huileuses, de dispersions du type des solutions ou dispersions du type lotion ou sérum, d'émulsions de consistance liquide ou semi-liquide du type lait, de suspensions ou émulsions, du type crème, de gel aqueux ou anhydre, de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain.

Les compositions selon l'invention peuvent également consister en des préparations solides constituant des savons ou des pains de nettoyage.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique. L'émulsionnant et le coémulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, les formes galéniques dédiées à une administration topique peuvent contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré et, par exemple, de 0,01 à 20 % du poids total de la composition. Ces adjuvants, selon leur nature peuvent être introduits dans la phase grasse et/ou dans la phase aqueuse.

Comme matières grasses utilisables dans l'invention, on peut citer les huiles minérales, comme par exemple, le polyisobutène hydrogéné et l'huile de vaseline, les huiles végétales, comme par exemple, une fraction liquide du beurre de karité, huile de tournesol et d'amandes d'abricot, les huiles animales, comme par exemple, le perhydrosqualène, les huiles de synthèse, notamment l'huile de Purcellin, le myristate d'isopropyle et le palmitate d'éthyl hexyle, les acides gras insaturés et les huiles fluorées comme par exemple les perfluoropolyéthers. On peut aussi utiliser des alcools gras, des acides gras, comme par exemple, l'acide stéarique et, comme par exemple, des cires, notamment de paraffine, carnauba et la cire d'abeilles. On peut aussi utiliser des composés siliconés comme les huiles siliconées et par exemple le cyclométhicone et diméthicone, les cires, les résines et les gommes siliconées.

Comme émulsionnants utilisables dans l'invention, on peut citer, par exemple, le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO® par la société HENKEL, le mélange de PEG-6/PEG-32/Glycol Stéarate vendu sous la dénomination de Tefose® 63 par société GATTEFOSSE, le PPG-3 myristyl éther, les émulsionnants siliconés, tels que le cétyldiméthicone copolyol et le mono- ou tristéarate de sorbitane, le stéarate de PEG-40, le monostéarate de sorbitane oxyéthyléné (200E).

Comme solvants utilisables dans l'invention, on peut citer les alcools inférieurs, notamment l'éthanol et l'isopropanol, le propylène glycol.

Une composition selon l'invention peut également contenir de façon avantageuse une eau thermale et/ou minérale, notamment choisie parmi l'eau de Vittel, les eaux du bassin de Vichy et l'eau de la Roche Posay.

Comme gélifiants hydrophiles, on peut citer les polymères carboxyliques tels que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305® par la société SEPPIC, les polysaccharides comme les dérivés cellulosiques tels que les hydroxyalkylcelluloses, et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Dans le cas d'une utilisation conforme à l'invention, par voie orale, on privilégie l'utilisation d'un support ingérable.

Le support ingérable peut être de diverses natures selon le type de composition considérée.

Conviennent ainsi notamment comme supports alimentaires ou pharmaceutiques des comprimés ou des tablettes, des suppléments oraux sous forme sèche et des suppléments oraux sous forme liquide.

Il peut par exemple s'agir de compléments alimentaires, dont la formulation peut être réalisée par les procédés usuels pour produire notamment des dragées, gélules, gels, émulsions, comprimés, capsules et hydrogels permettant une libération contrôlée.

En particulier, le microorganisme selon l'invention peut être incorporé dans toutes autres formes de compléments alimentaires ou d'aliments enrichis, par exemple des barres alimentaires ou des poudres, compactées ou non. Les poudres peuvent être diluées dans de l'eau, du soda, des produits laitiers ou dérivées du soja, ou être incorporées dans des barres alimentaires.

Le microorganisme, selon l'invention, peut être par ailleurs formulé avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires, sont connus dans ce domaine et ne font pas ici l'objet d'une description détaillée.

Conviennent notamment comme supports alimentaires ou pharmaceutiques, le lait, le yaourt, le fromage, les laits fermentés, les produits fermentés à base de lait, des glaces, des produits à base de céréales ou des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suspensions de bactéries liquides, des suppléments oraux sous forme sèche et des suppléments oraux sous forme liquide.

Un microorganisme conforme à l'invention peut être par ailleurs formulé avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires, sont connus dans ce domaine et ne font pas ici l'objet d'une description détaillée. Pour l'ingestion, de nombreuses formes de réalisation de compositions orales et notamment de compléments alimentaires sont possibles. Leur formulation est réalisée par les procédés usuels pour produire des dragées, gélules, gels, hydrogels à libération contrôlée, émulsions, comprimés, capsules.

Selon un mode de réalisation particulier, les microorganismes annexes considérés selon l'invention peuvent être formulés au sein de compositions sous une forme encapsulée de manière à améliorer significativement leur durée de survie. Dans un tel cas, la présence d'une capsule peut en particulier retarder ou éviter la dégradation du microorganisme au niveau du tractus gastro intestinal.

Le procédé de traitement cosmétique de l'invention peut être mis en oeuvre notamment en administrant par voie orale et/ou topique au moins une quantité efficace d'au moins un microorganisme conforme à l'invention.

L'administration par voie topique consiste à l'application externe sur la peau de compositions cosmétiques et/ou dermatologiques selon la technique d'utilisation habituelle de ces compositions.

A titre illustratif, le procédé cosmétique selon l'invention peut être mis en oeuvre par application topique, journalière par exemple, du microorganisme conforme à l'invention, qui peut être par exemple formulée sous forme de crèmes, gels, sérums, lotions, émulsions, laits démaquillants ou de compositions après-solaires.

Le procédé selon l'invention peut comprendre une application unique. Selon un autre mode de réalisation, l'application est répétée par exemple 2 à 3 fois quotidiennement sur une journée ou plus et généralement sur une durée prolongée d'au moins 4 jours, voire 1 à 15 semaines.

L'administration par voie orale consiste à ingérer en une ou plusieurs prises une composition orale telle que définie ci-dessus.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés avant leur mise en forme, dans l'ordre et dans des conditions facilement déterminées par l'homme de l'art.

Selon une variante, le procédé cosmétique comprend au moins une étape d'administration orale d'une quantité efficace d'au moins un microorganisme selon l'invention, de l'une de ses fractions, et au moins une étape d'administration topique d'une quantité efficace d'au moins un microorganisme selon l'invention ou de l'une de ses fractions.

Le procédé selon l'invention peut comprendre une administration unique.

Selon un autre mode de réalisation, l'administration est répétée par exemple 2 à 3 fois quotidiennement sur une journée ou plus et généralement sur une durée prolongée d'au moins 4 jours, voire 1 à 15 semaines, avec le cas échéant une ou plusieurs périodes d'interruption.

De plus il peut être envisagé des associations de traitement avec éventuellement des formes orales ou topiques afin de compléter ou renforcer l'activité du microorganisme tel que définie par l'invention.

Ainsi, on pourrait imaginer un traitement par voie topique avec une composition contenant un microorganisme conforme à l'invention, associé à une composition par voie orale ou topique contenant éventuellement un autre microorganisme, notamment probiotique ou d'autres probiotiques sous forme morte, vivante ou semi-active.

Les ingrédients sont mélangés, avant leur mise en forme, dans l'ordre et dans des conditions facilement déterminées par l'homme de l'art.

Les exemples ci-après sont présentés à titre illustratif et non limitatif du domaine de l'invention.

Dans ces exemples, le terme « ufc » désigne « unité formant une colonie ». C'est l'unité de mesure utilisée pour quantifier les bactéries vivantes.

Le *Lactobacillus paracasei* mis en oeuvre dans les compositions des exemples ci-après est le *Lactobacillus paracasei* ST11 NCC 2461 (CNCM I-2116).

### FIGURE

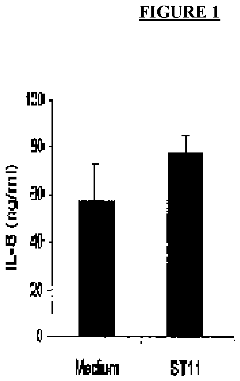

*Figure 1* : illustre le taux d'IL-8 mesuré à l'issu de l'exemple 2 dans les surnageants en soumettant le système de co-culture au milieu seul (Médium) ou additionné de *Lactobacillus paracasei* ST 11 (ST 11).

### EXEMPLE 1

### Exemples de compositions pour voie orale

**Exemple 1a : Stick poudre**

| **Principe actif** | |
|---|---|
| *Lactobacillus paracaseï ST11* | 10¹⁰ ufc |
| | |

| **Excipient** | |
|---|---|
| Gomme de xanthane | 0,8 mg |
| Benzoate de sodium | 0,2 mg |
| Maltodextrine | qsp 30g |

On peut prendre un stick par jour.

**Exemple 1b : Stick poudre**

| **Principe actif** | |
|---|---|
| *Lactobacillus paracaseï ST11* | 10¹⁰ ufc |
| | |

| **Excipient** | |
|---|---|
| Gomme de xanthane | 0,8 mg |
| Benzoate de sodium | 0,2 mg |
| Maltodextrine | qsp 30g |

On peut prendre un stick par jour.

**Exemple 1c : Capsules**

| **Principe actif** | **mg/capsule** |
|---|---|
| *Lactobacillus johnsonii* | 10⁸ ufc |
| Vitamine C | 60 |
| Stéarate de magnésium | 0,02 |

On peut prendre une à trois de ces capsules par jour.

**Exemple 1d : Formulation de type dragée**

| **Matières actives** | mg/dragée |
|---|---|
| *Lactobacillus paracaseï ST11* | 5,10⁸ ufc |
| Bifidobacterium longum | 5,10⁸ ufc |

| **Excipient du noyau de la dragée** | |
|---|---|
| Cellulose micro-cristalline | 70 |
| Encompress™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

Ce type de dragée peut être pris 1 à 3 fois par jour.

**Exemple 1e: Formulation de type dragée**

| **Matières actives** | mg/dragée |
|---|---|
| *Lactobacillus paracaseï ST11* | 10⁹ ufc |
| Lactobacillus johnsonii | 10⁹ ufc |

| **Excipient du noyau de la dragée** | |
|---|---|
| Cellulose micro-cristalline | 70 |
| Encompress™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvinylidone | 6 |
| Dioxyde de titane | 0,3 |
| Agent de coloration | 5 |

Ce type de dragée peut être pris 1 à 3 fois par jour.

### Exemples de composition pour voie topique

**Exemple 1f: Lotion pour le visage**

| | (% en poids) |
|---|---|
| Poudre de *Lactobacillus paracaseï ST11* | 5,00 |
| Poudre de *Lactobacillus jonhsonii* | 5,00 |
| Antioxydant | 0,05 |
| Isopropanol | 40,0 |
| Conservateur | 0,30 |
| Eau | qsp 100 % |

**Exemple 1 g : Gel pour le soin du visage**

| | (% en poids) |
|---|---|
| Poudre de *Lactobacillus paracaseï ST11* | 5,00 |
| Hydroxypropylcellulose (Klucel H® vendu par la société | 5,00 |
| HERCULES) | 1,00 |
| Vitamine E | 2,50 |
| Isopropanol | 40,00 |
| Conservateur | 0,30 |
| Eau | qsp 100 % |

**Exemple 1h : Lait pour le soin du visage**

| | (% en poids) |
|---|---|
| Poudre de *Lactobacillus paracaseï ST11* | 5,00 |
| Stéarate de glycérol | 1,00 |
| Alcool cétylstéarylique/alcool cétylstéarylique oxyéthyléné à 3 moles OE (Sinnovax AO® vendu par la société HENKEL | 3,00 |
| Alcool cétylique | 1,00 |
| Diméthicone (DC 200 Fluid® vendu par la société Dow Corning | 1,00 |
| Huile de vaseline | 6,00 |
| Myristate d'isopropyle (Estol IPM 1514® vendu par la société Unichema) | 3,00 |
| Glycérine | 20,00 |
| Conservateur | 0,30 |
| Eau | qsp 100 % |

**Exemple 1i : Crème pour le soin du visage**

| | (% en poids) |
|---|---|
| Poudre de Lactobacillus paracaseï ST11 | 5,00 |
| Glycérine | 2,0 % |
| Extrait de Vitreoscilla filiformis | 3,0 % |
| BHT | 0,05 % |
| POB méthyle | 0,1 % |
| POB propyle | 0,05 % |
| Eau | qsp 100 % |

**Exemple 1j : Gel pour le soin visage**

| | (% en poids) |
|---|---|
| Poudre de Lactobacillus paracaseï ST11 | 5,00 |
| Extrait de Vitreoscilla filiformis | 3,00 |
| Antioxydant | 0,05 |
| Vitamine C | 2,50 |
| Antioxydant | 0,05 |
| Isopropanol | 40,00 |
| Conservateur | 0,30 |
| Eau | qsp 100,00 % |

### EXEMPLE 2

Une lignée de cellules intestinales CaCO-2 est mise en culture sur des inserts de 10,5 mm (Becton Dickinson) à raison de 2 x 10⁵ cellules/puits. Ces inserts sont ensuite placés en culture dans une plaque de 12 puits (Nunc). Les cellules sont alors cultivées durant 21 jours à 37 °C dans 10 % CO₂ et dans du DMEM supplémenté avec 10 % FCS et 0,1 % de pénicilline/streptomycine (10 000 Ul/mL, Gibco BRL).

Les cellules mononucléaires de sang périphérique (leucocytes) humain sont purifiées à partir de poche de sang « buffy coats » par centrifugation à travers une colonne Ficoll-Hypaque 1077 (Pharmacia) puis sont resuspendues dans un milieu RPMI complet supplémenté de sérum humain AB (Gibco BRL). Les leucocytes (2 x 10⁶ cellules/mL) sont alors ajoutés dans le compartiment baso-latérale des cultures « trans-well » lorsque celles-ci présentent une couche confluente de cellules CaCO-2 qui ont été préalablement lavées de leur milieu.

Les co-cultures ainsi établies sont stimulées en ajoutant 1 x 10⁷ CFU/mL de probiotiques au niveau de la surface apicale de la mono-couche de cellules épithéliales (CaCO-2). Le système est ensuite incubé durant 16h à 37 °C/5 % CO₂.

Afin d'éviter la croissance des bactéries, 150 µg/mL de Gentamicine sont ajoutés au milieu après 4h d'incubation (Figure 1).

En fin d'incubation (16h) le milieu se trouvant dans le compartiment baso-latérale est prélevé pour être testé.

Une première série d'analyse consiste à analyser la présence de marqueurs d'inflammation ou d'immuno-régulation (IL-8, TGF-β).

La figure 1 illustre la mesure du marqueur pro-inflammatoire IL-8 produit dans les milieux des co-cultures stimulées avec le probiotique *Lactobacillus paracaseï:*

On n'observe pas d'augmentation significative d'IL-8 dans le milieu stimulé par *Lactobacillus paracaseï* ST 11 par rapport au contrôle, milieu non stimulé par des probiotiques.

Ces résultats confirment le caractère non-pro-inflammatoire du probiotique sélectionné. De hauts niveaux d'expression de TGF-β ont, de plus, été détectés dans ce système.

## Revendications

1. Utilisation cosmétique d'une quantité efficace d'au moins un microorganisme probiotique de l'espèce *Lactobacillus paracasei,* comme agent pour prévenir une irritation cutanée induite par une ou des agression(s) externe(s) choisies parmi :
- des sollicitations mécaniques ; ou
- des déséquilibres thermiques ou climatiques.

2. Utilisation selon la revendication précédente, dans laquelle :
- les sollicitations mécaniques sont choisies parmi: les frottements, les chocs, l'abrasion, l'arrachement de la surface, les projections de poussières, les projections de particules, le rasage, l'épilation ;
- les déséquilibres thermiques ou climatiques sont choisis parmi : le froid, la sécheresse.

3. Utilisation selon la revendication 1, **caractérisée en ce que** l'irritation cutanée est induite par au moins une condition choisie parmi : l'action de produits chimiques, de composés susceptibles de provoquer une irritation de la peau, de la température, du climat, de la pollution atmosphérique ou des frottements.

4. Utilisation selon l'une quelconque des revendication précédentes, pour prévenir et/ou traiter les troubles cutanés de type irritation consécutifs à une exposition aux polluants atmosphériques.

5. Utilisation selon l'une quelconque des revendication précédentes, pour prévenir et/ou traiter les troubles cutanés de type irritation générés par un stress oxydatif initié par un oxydant environnemental choisi parmi : l'oxygène, l'ozone, les oxydes d'azote et le souffre.

6. Utilisation selon l'une quelconque des revendications précédentes, **caractérisée en ce que** ledit microorganisme probiotique est destiné à prévenir et/ou diminuer l'effet irritant d'une composition cosmétique ou dermatologique contenant un ou plusieurs composés susceptibles de provoquer une irritation de la peau.

7. Utilisation selon l'une quelconque des revendications précédentes, pour prévenir et/ou traiter et/ou diminuer une irritation cutanée non allergique.

8. Utilisation selon l'une quelconque des revendications précédentes, pour prévenir et/ou traiter une dermatite irritante de contact non allergique.

9. Utilisation selon l'une quelconque des revendications précédentes, **caractérisée en ce que** le microorganisme est le *Lactobacillus paracasei* CNCM 1-2116.

10. Utilisation selon l'une quelconque des revendications précédentes, dans laquelle ledit microorganisme est mis en oeuvre à raison de 0,0001 à 20 % en poids, en particulier de 0,001 à 15 % et plus particulièrement de 0,01 à 10 % en poids par rapport au poids total de la composition le contenant.

11. Utilisation selon l'une quelconque des revendications précédentes, dans laquelle ledit microorganisme est mis en oeuvre par voie orale.

12. Utilisation selon la revendication 1, où le dit microorganisme probiotique de l'espèce *Lactobacillus paracasei* est sous la forme d'une poudre lyophilisée, d'un surnageant de culture et/ou sous une forme concentrée.

13. Composition comprenant une quantité efficace d'au moins un microorganisme probiotique de l'espèce *Lactobacillus paracasei,* , comme agent destiné à être utilisé pour prévenir une irritation cutanée induite par des agents irritants.

14. Composition selon la revendication précédente, **caractérisée en ce que** les agents irritants sont choisis parmi : les détergents, les acides, les bases, les oxydants, les réducteurs, les solvants concentrés, les gaz et fumées toxiques.

## Patentansprüche

1. Kosmetische Verwendung einer wirksamen Menge mindestens eines probiotischen Mikroorganismus der Spezies *Lactobacillus paracasei* als Mittel zur Vorbeugung einer Hautreizung, die durch einen äußeren Angriff bzw. äußere Angriffe hervorgerufen wird, ausgewählt aus:
- mechanischen Beanspruchungen oder
- thermischen oder klimatischen Ungleichgewichten.

2. Verwendung nach dem vorhergehenden Anspruch, wobei:
- die mechanischen Beanspruchungen ausgewählt sind aus: Reibung, Stößen, Abrasion, Ablösen der Oberfläche, aufgewirbeltem Staub, herumfliegenden Partikeln, Rasieren, Epilation;
- die thermischen oder klimatischen Ungleichgewichte ausgewählt sind aus: Kälte, Trockenheit.

3. Verwendung nach Anspruch 1, **dadurch gekennzeichnet, dass** die Hautreizung durch mindestens eine Bedingung hervorgerufen wird, ausgewählt aus: der Einwirkung chemischer Produkte, Verbindungen, die eine Reizung der Haut hervorrufen können, Temperatur, Klima, Luftverschmutzung oder Reibung.

4. Verwendung nach einem der vorhergehenden Ansprüche zur Vorbeugung und/oder Behandlung von Hautbeschwerden des Typs einer Reizung nach einer Exposition gegenüber Luftschadstoffen.

5. Verwendung nach einem der vorhergehenden Ansprüche zur Vorbeugung und/oder Behandlung von durch einen oxidativen Stress erzeugten Hautbeschwerden des Typs einer Reizung, die durch ein Umwelt-Oxidans ausgelöst werden, ausgewählt aus: Sauerstoff, Ozon, Stickstoff- und Schwefeloxiden.

6. Verwendung nach einem der vorhergehenden Ansprüche, **dadurch gekennzeichnet, dass** der probiotische Mikroorganismus dazu dient, die reizende Wirkung einer kosmetischen oder dermatologischen Zusammensetzung, die eine oder mehrere Verbindungen enthält, die eine Reizung der Haut hervorrufen können, zu verhindern und/oder zu verringern.

7. Verwendung nach einem der vorhergehenden Ansprüche zur Vorbeugung und/oder Behandlung und/oder Verringerung einer nicht-allergischen Hautreizung.

8. Verwendung nach einem der vorhergehenden Ansprüche zur Vorbeugung und/oder Behandlung einer nicht-allergischen reizenden Kontaktdermatitis.

9. Verwendung nach einem der vorhergehenden Ansprüche, **dadurch gekennzeichnet, dass** der Mikroorganismus *Lactobacillus paracasei* CNCM I-2116 ist.

10. Verwendung nach einem der vorhergehenden Ansprüche, wobei der Mikroorganismus zu 0,0001 bis 20 Gew.-%, insbesondere zu 0,001 bis 15 Gew.-% und ganz besonders zu 0,01 bis 10 Gew.-%, bezogen auf das Gesamtgewicht der Zusammensetzung, die ihn enthält, eingesetzt wird.

11. Verwendung nach einem der vorhergehenden Ansprüche, wobei der Mikroorganismus oral angewendet wird.

12. Verwendung nach Anspruch 1, wobei der probiotische Mikroorganismus der Spezies *Lactobacillus paracasei* in Form eines lyophilisierten Pulvers, eines Kulturüberstands und/oder in konzentrierter Form vorliegt.

13. Zusammensetzung, umfassend eine wirksame Menge mindestens eines probiotischen Mikroorganismus der Spezies *Lactobacillus paracasei* als Mittel für die Verwendung zur Vorbeugung einer durch Reizstoffe hervorgerufenen Hautreizung.

14. Zusammensetzung nach dem vorhergehenden Anspruch, **dadurch gekennzeichnet, dass** die Reizstoffe ausgewählt sind aus: Detergentien, Säuren, Basen, Oxidantien, Reduktionsmitteln, konzentrierten Lösungsmitteln, toxischen Gasen und Dämpfen.

## Claims

1. Cosmetic use of an effective amount of at least one probiotic microorganism of the species *Lactobacillus paracasei* as agent for preventing cutaneous irritation induced by one or more external aggressions(s) chosen from:
- mechanical stresses; or
- thermal or climatic imbalances.

2. Use according to the preceding claim, wherein:
- the mechanical stresses are chosen from: friction, impacts, abrasion, surface tearing, thrown up dust, thrown up particles, shaving, hair removal;
- the thermal or climatic imbalances are chosen from: cold, dryness.

3. Use according to Claim 1, **characterized in that** the cutaneous irritation is induced by at least one condition chosen from: the action of chemical products, of compounds liable to cause skin irritation, of the temperature, of the climate, of atmospheric pollution or of friction.

4. Use according to any one of the preceding claims, for preventing and/or treating cutaneous disorders of irritation type following exposure to atmospheric pollutants.

5. Use according to one of the preceding claims for preventing and/or treating cutaneous disorders of irritation type generated by oxidative stress initiated by an environmental oxidant chosen from: oxygen, ozone, nitrogen oxides and sulfur.

6. Use according to any one of the preceding claims, **characterized in that** said probiotic microorganism is intended to prevent and/or reduce the irritant effect of a cosmetic or dermatological composition containing one or more compounds liable to cause skin irritation.

7. Use according to any one of the preceding claims, for preventing and/or treating and/or reducing non-allergic cutaneous irritation.

8. Use according to any one of the preceding claims, for preventing and/or treating a non-allergic irritant contact dermatitis.

9. Use according to any one of the preceding claims, **characterized in that** the microorganism is *Lactobacillus paracasei* CNCM 1-2116.

10. Use according to any one of the preceding claims, wherein said microorganism is employed at an amount of 0.0001 to 20% by weight, in particular 0.001 to 15% and more particularly 0.01 to 10% by weight relative to the total weight of the composition containing same.

11. Use according to any one of the preceding claims, wherein said microorganism is employed orally.

12. Use according to Claim 1, wherein said probiotic microorganism of the species *Lactobacillus paracasei* is in the form of a lyophilized powder, of a culture supernatant and/or in concentrated form.

13. Composition comprising an effective amount of at least one probiotic microorganism of the species *Lactobacillus paracasei* as agent intended to be used for preventing cutaneous irritation induced by irritants.

14. Composition according to the preceding claim, **characterized in that** the irritants are chosen from: detergents, acids, bases, oxidants, reducing agents, concentrated solvents, gases and toxic fumes.
